# EUROPEAN PATENT APPLICATION

(11) **EP 2 703 395 A1**
(43) Date of publication of application: **05.03.2014**
(21) Application number: 12182758.8
(22) Date of filing: 03.09.2012
(51) Int. Cl.: C07D 307/50

(54) **Process for the production of furan derivatives**

(71) Applicant: Annikki GmbH, 8020 Graz (AT)
(72) Inventor: Mihovilovic, Marko, 2380 Perchtoldsdorf (AT); Schön, Michael, 1140 Wien (AT); Hölbling, Johanna, 4880 St. Georgen/Attergau (AT)
(74) Representative: Schwarz, Albin

(57) **Abstract**

A process for the production of furan derivatives from carbohydrates in the presence of an acidic catalyst wherein N-methylpyrrolidone is used as a solvent.

## Description

The present invention relates to the production of furan derivatives from carbohydrates in the presence of an acidic catalyst.

Numerous processes for the production of furan derivatives from carbohydrates are known.

In such processes several different acidic catalysts are in use: classical inorganic acids, see e.g. Chheda, J. N.; Roman-Leshkow, Y.; Dumesic, J. A. Green Chem. 2007, 9, 342-350: organic acids (e.g. oxalic acid), H-form zeolites, transition metal ions, see e.g. Young, G.; Zhang, Y.; Ying, J. Y. Angew. Chem. Int. Ed. 2008, 47, 9345-9348; Tyrlik, S. K.; Szerszen, D.; Olejnik, M.; Danikiewicz, W. Carbohydr. Res. 1999, 315, 268-272; solid metal phosphates, see e.g. Asghari, F. S.; Yoshida, H. Carbohydr. Res. 2006, 341, 2379-2387; strong acid cation exchange resins, see e.g. Villard, R.; Robert, F.; Blank, I.; Bernardinelli, G.; Soldo, T.; Hofmann, T. J. Agric. Food Chem. 2003, 51, 4040-4045.

In such processes water as a solvent was intensively investigated as a green solvent. While the system containing biomass and water represents a green approach, on the other hand temperatures of > 300 °C and pressures at around 20 MPa are required to achieve acceptable yields, see e.g. Qi, X.; Watanabe, M.; Aida, T. M.; Smith Jr., R. S. Cat. Commun. 2008, 9, 2244-2249.

A furan derivative which may be produced from carbohydrates in the presence of an acidic catalyst includes 5-hydroxymethylfurfural (HMF). Processes for the production of HMF are known. In aqueous solution, homogeneous and heterogenous acid catalysts can be used to produce HMF starting from carbohydrates. The achieved yields of HMF are between 30 to 60% depending on the carbohydrate source and the exact reaction conditions. Drawbacks when using water as a reaction solvent are the formation of byproducts, especially levulinic acid and insoluble humins. Furthermore these reactions must be carried out at very harsh conditons up to 300°C and 27 MPa, see e.g. Bicker, M., Kaiser, D., Ott, L., Vogel, H., J. of Supercrit. Fluids 2005, 36,118-126; Szmant, H. H., Chundury, D. D., J. Chem. Techn. Biotechnol. 1981, 31, 135-145; Srokol, Z., Bouche, A.-G., van Estrik, A., Strik, R. C. J., Maschmeyer, T., Peters, J. A., Carbohydr. Res. 2004, 339, 1717-1726). A flow process under supercritical conditions starting from glucose was described by Aida, T. A.; Sato, Y.; Watanabe, M.; Tajima, K.; Nonaka, T.; Hattori, H.; Arai, K. J. of Supercrit. Fluids, 2007, 40, 138-388.

Organic solvents may be suitable solvents also in the preparation of HMF, but a critical limitation is that such solvents may be difficult to separate from the product HMF, see e.g. Bao, Q.; Qiao, K.; Tomido, D.; Yokoyama, C. Catal. Commun. 2008, 9, 1383-1388; Halliday, G. A.; Young Jr., R. J.; Grushin, V. V. Org. Lett. 2003, 5, 2003-2005. Furthermore, previously employed organic solvents for HMF are not inert to subsequent reaction conditions to form HMF derivatives when the solvent is not separated from the HMF intermediate. Commonly employed organic solvents for the formation of HMF from carbohydrates are DMSO and dimethylformamide (DMF). In comparison to water as a solvent the reaction of carbohydrates to furan derivates can be carried out at lower temperatures (80-140°C) and even with higher yields of HMF (up to 95% in DMSO) in short reaction times (30 min - 2 h), see e.g. Halliday, G. A., Young Jr., R. J., Grushin, V. V., Org. Lett. 2003, 5, 2003-2005; WO 2009/076627 A2. Nevertheless, these polar organic solvents promote the dehydratization of fructose (and other carbohydrates) to HMF (and derivatives), as e.g. DMSO is also acting as a catalyst, see Amarasekara, A. S.; Williams, L. D.; Ebede, C. C. Carbohydr. Res. 2008, 343, 3021-3024.

Reaction mixtures of water/DMSO or water/toluene are known and also applied to continuous extraction, see e.g. Chheda, J. N., Roman-Leshkov, Y., Dumesic, J. A., Green Chem. 2007, 9, 342-350. The reaction takes between 4 to 6 hours at 140-180°C, resulting in 80% HMF yield at best.

Ionic liquids can act as neutral solvents but also as Brønsted acids and they can even be immobilized on silica gel, e.g. as disclosed in Bao, Q.; Qiao, K.; Tomido, D.; Yokoyama, C. Catal. Commun. 2008, 9, 1383-1388, but the separation of HMF and the ionic liquid still remains difficult.

All the known processes have drawbacks, e.g. harsh conditions if water is used as a solvent, or isolation issues if highly polar solvents such as DMF or DMSO are used which may result in high energy consumpting processes and/or which may result in insufficient purity/yield.

It was now found surprisingly that reaction conditions can be tempered which in consequence may increase the process efficiency, e.g. in terms of energy consumption, product purity, yields, suppression of humeric polymer production, if a specific organic solvent is used in the production of furan derivatives from carbohydrates under acidic catalysis.

In one aspect, the present invention provides a process for the production of furan derivatives from carbohydrates in the presence of an acidic catalyst, characterized in that N-methylpyrrolidone is used as a solvent.

A process provided by the present invention is herein also designated as "process of (according to) the present invention".

The use of N-methylpyrrolidone (NMP) as a solvent according to the present invention includes that NMP is used as the (sole) reaction solvent and that NMP is used as a reaction co-solvent, e.g. NMP may be used alone or in combination with other inorganic or organic solvent.

In another aspect the present invention provides a process according to the present invention, which is characterized in that N-methylpyrrolidone is used as the reaction solvent; and in another aspect that N-methylpyrrolidone is used as a reaction co-solvent.

In process of the present invention, a carbohydrate preferably is a sugar, e.g. a sugar which may be obtained from biomass, more preferably a sugar which may be dehydrated to obtain a furan derivative. Such sugars e.g. include C5 and C6 sugars, preferably C6 sugars, such as fructose, and natural and synthetic sugars, e.g. natural sugars, such as D-(-)-fructose.

In another aspect, the present invention provides a process for the production of a furan derivative from a sugar, e.g. comprising dehydratation of a sugar, in the presence of an acidic catalyst in wherein N-methylpyrrolidone is used as a solvent, e.g. or co-solvent.

In process of the present invention, a furan derivative is preferably a furan substituted by an aldehyde, e.g. and further substituted by a hydroxy group, such as 5-hydroxymethylfurfural (HMF), e.g. of formula

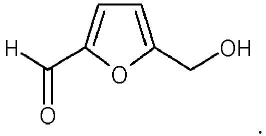

In another aspect, the present invention provides a process for the production of 5-hydroxymethylfurfural from a sugar, e.g. comprising dehydratization of a sugar, in the presence of an acidic catalyst, wherein N-methylpyrrolidone is used as a solvent, e.g. or co-solvent.

A process of the present invention may be carried out as a batch process or as a continuous process, optionally under microwave irridation.

In another aspect the present invention provides a process of the present invention which is characterized in that the process is carried out as a batch process or as a continuous process, optionally under microwave irridation.

In a process of the present invention an acidic catalyst is used. Useful acidic catalysts are listed in the preamble of the present application and include a catalyst which is homogenous or a catalyst which is heterogenous. In one preferred embodiment of the present invention a homogenous catalyst, e.g. an inorganic acid such as HCl, H₂SO₄ in another preferred embodiment of the present invention a heterogenous catalyst, e.g. a Montmorillonite, in particular Montmorillonite KSF^{®}, or an Amberlite, in particular Amberlite 15^{®} is used.

In another aspect the present invention provides a process of the present invention which is characterized in that a homogenous catalyst, e.g. an inorganic acid such as HCl, H₂SO₄, or a heterogenous catalyst, e.g. a Montmorillonite, in particular Montmorillonite KSF^{®}, or an Amberlite, in particular Amberlite 15^{®} is used.

In a process of the present invention, the reaction temperature may be far below 300°C, e.g. in a range of 100 to 220°C, preferably from 125 to 200°C, more preferably from 140°C to 170°C.

The reaction time of a reaction according to the present invention is dependent from the process used. In general the reaction time, however, is surprisingly short, e.g. from 30 seconds to 20 minutes, preferably from 1 minute to 10 minutes, more preferably from 2 to 6 minutes.

### Short description of the Figures (Fig. 1 to Fig. 7)

Fig. 1 shows the results of a time screening in an experiment performed in batch with sulphuric acid as a catalyst according to 5.1.1
Fig. 2 and Fig. 3 show results of experiments performed in the microwave with sulphuric acid as catalyst according to 5.2.1.
Fig. 4 and Fig. 5 show results of experiments performed in the microwave with hydrochloric acid as catalyst according to 5.2.2.
Fig. 6 shows results of a time screening in an experiment performed in the microwave with Montmorillonite KSF^{®} as a catalyst according to 5.2.3.
Fig. 7 shows results of experiments performed in flow (continous process) with hydrochloric acid as a catalyst according to 5.3.2.

In the following examples all temperatures are in degrees Celsius (°C).

The following abbreviations are used (herein and in the examples):
- aqu.: aqueous
- cons.: consumption
- HMF: 5-Hydroxymethylfurfural
- h: hour(s)
- IC: Interconversion
- LA: Levulinic acid
- NMP: N-Methylpyrrolidone
- RI: Refractive Index (Detector)
- SILP: Silica-supported Ionic Liquid Particles

- CMF: 5-Chloromethylfurfural
- EtOAc: Ethyl acetate
- HPLC: High performance liquid chromatography
- IL: Ionic liquid(s)
- min: minute(s)
- PDA: Photo Diode Array (Detector)
- rt: room temperature
- Temp: Temperature
- TFA: Trifluoroacetic acid

### 2. Overview

Dehydration reactions from fructose to HMF were carried out examining a variety of reaction conditions, using standard batch chemistry, but also microwave-assisted heating methods and continuous flow chemistry, as depicted in the Reaction Scheme I below. Surprisingly, NMP was found to be a most efficient solvent for this conversion compared to reported systems, in particular suitable for processes operating under homogeneous as well as heterogeneous catalysis and under both, microwave and flow chemistry conditions.

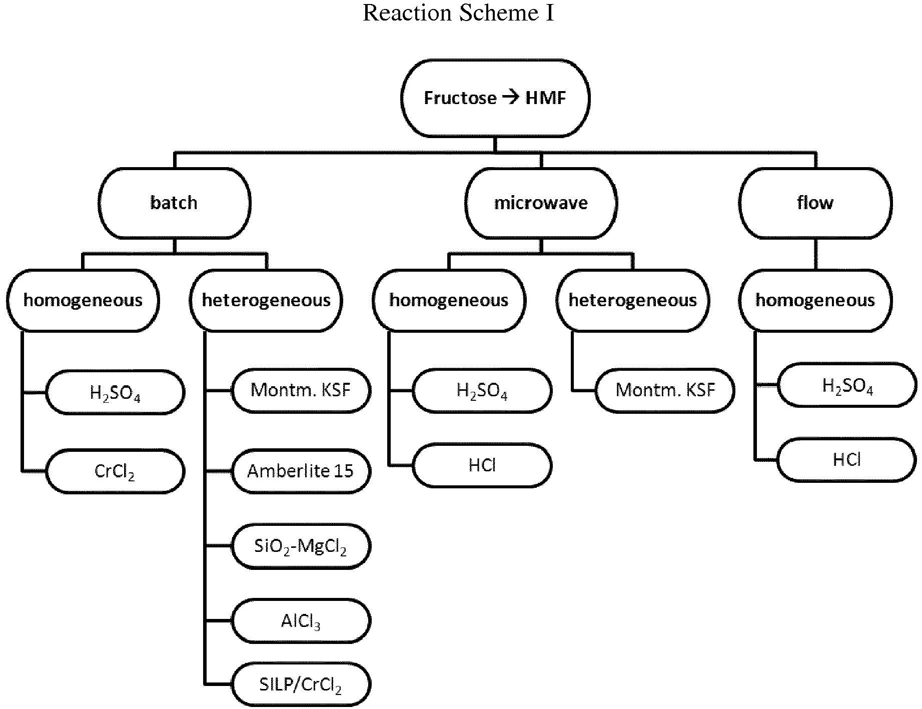

### 3. Materials and methods

All reactions and samples were prepared as double experiments.

### 3.1 Materials

D-(-)-Fructose, and 3-hydroxybenzyl alcohol were purchased from Fluka. Levulinic acid was used from Aldrich for calibration of by-product formation. The catalysts Montmorillonite KSF^{®} and Amberlite 15^{®} were purchased from Fluka; chromium-(II)-chloride, aluminium trichloride and silica were delivered by Aldrich; magnesium chloride was supplied by Merck and hydrochloric acid, as well as sulphuric acid were bought from Busetti and diluted to the desired concentrations. Methanol was used from Donauchem and anhydrous NMP was supplied by Merck.

### 3.1.1 Synthesis of HMF

For reference purposes, HMF was prepared in small analytical samples. Fructose was reacted to CMF according to Hamad, K., Yoshihara, H., Suzukamo, G., Chem. Lett. 1982, 617-618 and further converted to HMF via nucleophilic substitution:
CMF (2 g, 13.8 mmol) and deionized water (20 mL) were filled into a microwave vial and heated to 80°C for 3 min. The solution obtained was extracted three times with EtOAc, the combined organic layers were washed with aqu. saturated NaHCO₃ solution and dried over anhydrous Na₂SO₄. After filtration of the solid, the solvent obtained was evaporated under reduced pressure to give crude product, which was further purified via chromatography (SiO₂, CH₂Cl₂: CH₃OH = 95 : 5). Pure HMF in the form of a light yellow oil (1.12 g, 8.85 mmol, 64% of theory) was obtained which solidified upon storage at -30°C.

### 3.1.2 Synthesis of SiO₂-MgCl₂

SiO₂-MgCl₂ was prepared according to Yasuda, M.; Nakamura, Y.; Matsumoto, J.; Yokoi, H. Shiragami, T. Bull. Chem. Soc. Jpn. 2011, 84, 416-418. For that 1 g of silica (SiO₂) and 0.8 g of MgCl₂·6H₂O were added to 40 mL of CH₃OH. The mixture obtained was left standing for 3 h at rt and solvent was removed under vacuum. SiO₂-MgCl₂ was obtained and was dried under reduced pressure at 50°C for 24 h.

### 3.1.3 Synthesis of SILPs

The SILP catalyst was synthesized in a two-step reaction starting from N-methylimidazole. Quaternization was performed using a literature-known procedure (Fu, S.-K.; Liu, S.-T. Synth Commun. 2006, 36, 2059-2067) at 245°C in the microwave for 45 sec according to Reaction Scheme II below:

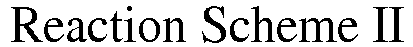

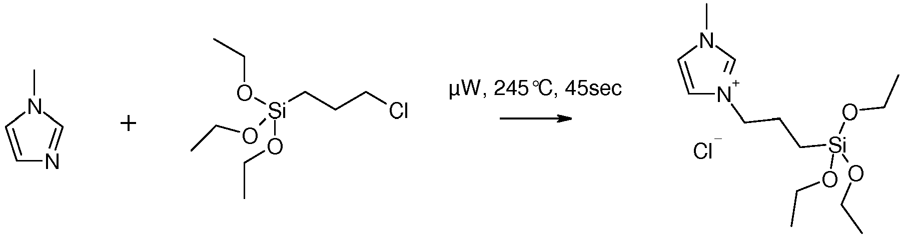

For immobilization of the ionic liquid moiety onto dry silica gel, a mixture of triethoxysilyl derivative (50% m/m, calculated to SiO₂) and dry silica gel were dissolved in dry CHCl₃ (100 mL per 10 g SiO₂) and reacted for 24 h at 70°C. The solid residue obtained was filtered, washed with CHCl₃ and dried under reduced pressure to give SILP catalyst with approximately 16wt-% IL loading. For that see Reaction Scheme III below:

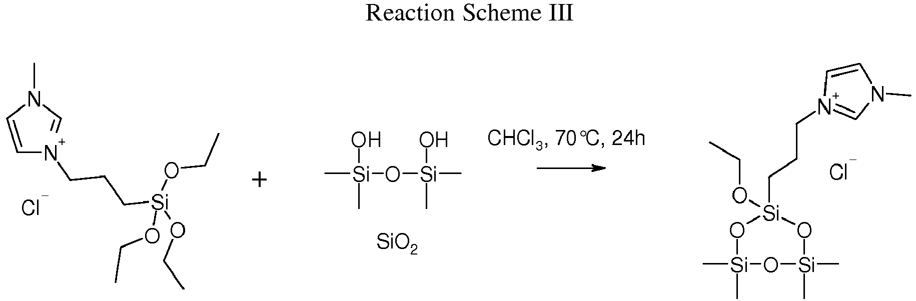

### 3.2 Batch reactions

If not stated otherwise, all batch reactions were carried out in 4 mL glass vials with screw caps and heated in appropriate aluminium heating blocks maintaining the desired temperatures.

### 3.3 Microwave batch reactions

Microwave reactions in batch were performed using a Biotage Initiator Sixty laboratory microwave, equipped with an autosampler allowing sequential reactions. Absorption level was set to the highest possible setting and maximum irradiation power was automatically regulated to 400 W.

### 3.4 Stopped flow microwave and continuous flow reactions

Stopped flow reactions to optimize for a semi-continuous microwave process were carried out in a CEM^{®} Discover system with the CEM^{®} Voyager upgrade and an external pressure sensor for reactions in small vials.

Reactions in continuous flow were performed in the cartridge-based reactor system X-Cube from ThalesNano^{®}, supplied with a Gilson^{®} GX-271 autosampler to allow for automated product collection. Two quartz sand cartridges (CatCart^{®}, 70 x 4 mm) were installed as a reaction bed.

Alternatively, a PFA (perfluoroalkoxy alkane) capillary (0.8 mm inner diameter, 1.6 mm outer diameter) was wrapped around an aluminium cylinder which was heated to the desired temperature. Starting materials were pumped using a Shimadzu LC-10AD HPLC pump at the appropriate flow rate. Exact volumes (column: 16.0 mL, pre- and post-volume: 1.0 mL each) were evaluated using a defined flow rate, a digital stop watch and pure solvent only. The exact setup is shown in the following Reaction Scheme IV:

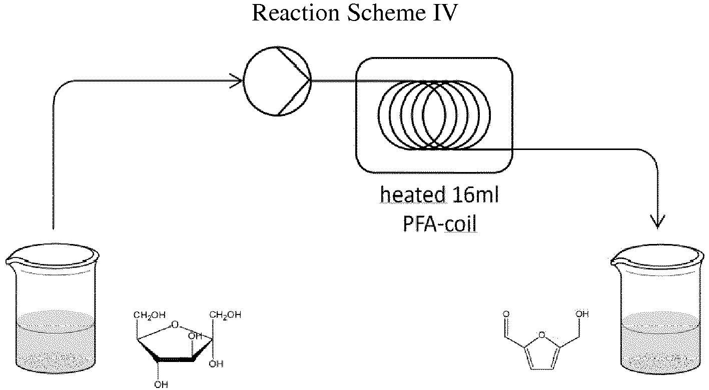

### 3.5 Analysis

Reaction analysis was performed by HPLC on a Thermo Scientific® Surveyor Plus System equipped with a PDA Plus and RI detector or a Shimadzu® Nexera system equipped with the same detectors. For the separation, an ion-exclusion column from Phenomenex® (Rezex RHM-Monosaccharide H+ (8%), 150 x 7.8 mm, sulfonated styrene divinyl benzene matrix, hydrogen ionic form) was used running on HPLC-grade water / 0.1% HPLC-grade TFA as a mobile phase. The run temperature was adjusted to 85°C and run time was optimized to 25 minutes. Product quantification was achieved by an internal standard method and RI detection, discrete PDA wavelengths were set to 200 nm, 254 nm and 280 nm for further evaluation of the reactions.

### 4. General procedures

### 4.1. Preparation of HPLC samples

To allow for accurate HPLC quantification, all reaction samples were diluted to a maximum carbohydrate concentration of 2 mg/mL. A sample (22 µL) was dissolved in deionized water (978 µL), internal standard (100 µL 3-hydroxybenzyl alcohol) was added and the sample was mixed thoroughly. Solid residues were separated by centrifugation (5 min, 20.000 g) or filtration (Phenex PTFE, 4 mm, 0.2 µm) and quantification of carbohydrates and products was achieved via refractive index detection on HPLC.

For reaction samples having a different concentration, the dilution values were adapted appropriately to ensure not to exceed a maximum concentration of 2 mg/mL.

### 4.2 GP1 - Fructose dehydratization in batch

As a standard procedure, fructose (100 mg, 555 µmol) and catalyst were loaded into a glass vial, equipped with a magnetic stirring bar. Freshly distilled NMP (1 mL) was added and the reaction solution obtained was stirred at the selected temperature.

### 4.3 GP2 - Fructose dehydratization in the microwave

Fructose (100 mg, 555 µmol) and catalyst were loaded into a microwave vial (0.5 - 2.0 mL size) and equipped with a magnetic stirring bar. NMP (1 mL) was added and irradiation power was automatically adjusted by the microwave's regulation algorithms. An appropriate cooling rate was achieved by supplying pressurized air with a pressure of at least 6 bar to directly cool the microwave vessel.

### 4.4 GP3 - Fructose dehydratization in stopped flow microwave reactor systems

Fructose stock solution (1 mL; c = 100 mg/mL in NMP) and hydrochloric acid (100 µL; c = 1 mol/L) were charged into a microwave vial equipped with a magnetic stirring bar. After sealing the vial with a snap-cap, the reaction solution was heated adjusting the desired reaction temperature and duration. To ensure for a rapid heating process, coupling power was adjusted according to the following Table 1:

**Table 1**

| **Temperature (°C)** | **Power Rating (W)** | **Temperature(°C)** | **Power Rating (W)** |
|---|---|---|---|
| 100 | 50 | 180 | 125 |
| 125 | 65 | 200 | 140 |
| 150 | 100 | 220 | 160 |

An appropriate cooling rate was achieved by supplying pressurized air with a pressure of at least 6 bar to directly cool the microwave vessel.

### 4.5 GP4 - Fructose dehydratization in cartridge-based reactor systems

Fructose stock solution (c = 100 mg/mL in NMP) was mixed with hydrochloric acid (c = 1 mol/L) and supplied to the reactor system via reagent pump A. During the heating process, several pre-samples were collected to guarantee for a stable temperature and flow rate. Reaction temperatures were selected at 150°C, 180°C and 200°C, pressure during the reactions was regulated to 40 bar, flow rates were adjusted from 0.2 mL/min to 0.6 mL/min and collected fractions to 2.5 mL each.

### 5. Results & Discussion

### 5.1 Experiments performed in batch

### 5.1.1 Sulphuric acid as a catalyst

To evaluate the dehydration properties of sulphuric acid in NMP, a variety of temperatures and acid concentrations was examined. Samples were prepared according to GP1 using either 100 µL 1N sulphuric acid solution or 10 µL concentrated sulphuric acid as catalyst, leading to the following results set out in Table 2 below:

**Table 2**

| **Catalyst** | **Temp.** | **Reaction time** | **Fructose cons.** | **HMF yield** | **HMF selectivity** | **LA yield** |
|---|---|---|---|---|---|---|
| 1N H₂SO₄ | 100°C | 3h | 69% | 45% | 65% | < 1% |
| 1N H₂SO₄ | 120°C | 4h | 95% | 77% | 81% | < 1% |
| 1N H₂SO₄ | 150°C | 15 min | 98% | 88% | 90% | < 1% |
| 1N H₂SO₄ | 180°C | 10 min | 100% | 85% | 85% | < 1% |
| H₂SO₄ conc. | 120°C | 45 min | 98% | 85% | 90% | < 1% |
| H₂SO₄ conc. | 150°C | 10 min | 100% | 90% | 90% | < 1% |
| H₂SO₄ conc. | 180°C | 5 min | 100% | 82% | 82% | < 1% |

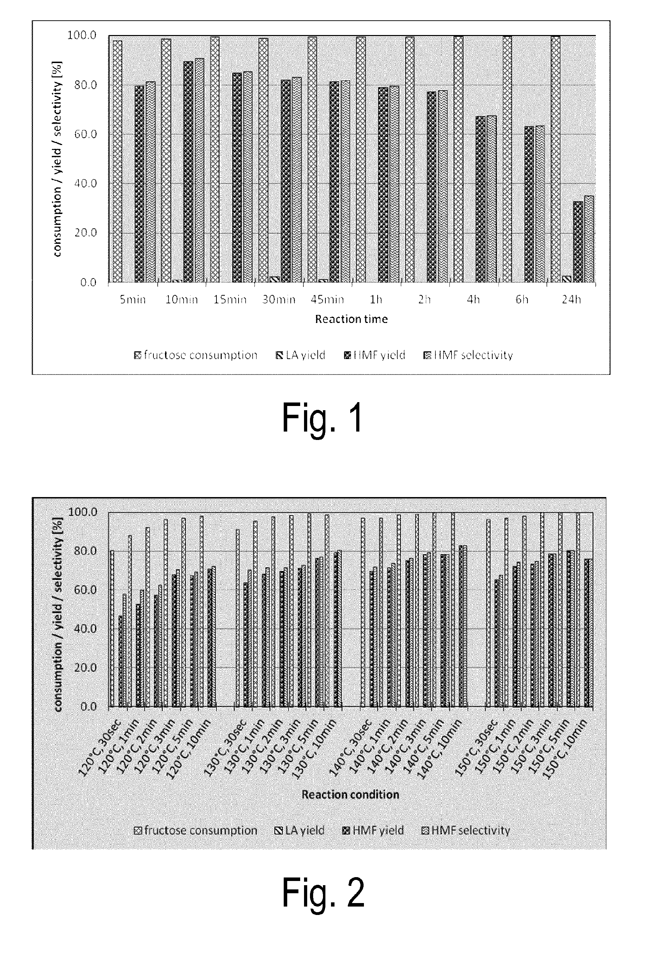

Formation of black, insoluble polymers and humins were not observed under the applied, optimal conditions. To characterize the exact progress of the dehydratization, time screenings were performed. A representative time course is shown in Fig. 1 (H₂SO₄ conc., 150°C).

### 5.1.2 Chromium-(II)-chloride as a catalyst

As Zhao, H.; Holladay, J. E.; Brown, H.; Zhang, Z. C. Science 2007, 316, 1597-1600 reported chromium-(II)-chloride to be a very efficient catalyst for fructose dehydratization, the effect of CrCl₂ in NMP was also investigated. The experiments were carried out according to GP1. Interestingly, all samples showed moderate HMF yields, but significantly higher amounts of black tar in each sample, e.g. as set out in Table 3 below:

**Table 3**

| **Catalyst amount** | **Temp. (°C)** | **Reaction time** | **Fructose cons.** | **HMF yield** | **HMF selectivity** | **LA yield** |
|---|---|---|---|---|---|---|
| 10 mg CrCl₂ | 100 | 3h | 86% | 51% | 59% | <1% |
| 10 mg CrCl₂ | 150 | 3h | 100% | 39% | 39% | <1% |

### 5.1.3 Montmorillonite KSF^{®} as a catalyst

Apart from homogeneous catalysts, also heterogeneous catalysts were successfully tested with NMP. Montmorrilonite KSF^{®}, a clay catalyst, was applied in different amounts using GP1 as working procedure. Reaction time was set to 3 h in all experiments to facilitate comparison of yields and black tar formation. Best conditions gave 61% HMF yield at 63% HMF selectivity, see e.g. Table 4 below:

**Table 4**

| **Catalyst amount** | **Temp. (°C)** | **Fructose cons** | **HMF yield** | **HMF selectivity** | **LA yield** | **Black tar** |
|---|---|---|---|---|---|---|
| 1 mg | 120 | 37% | 11% | 31% | <1% | no |
| 3 mg | 120 | 54% | 20% | 38% | <1% | no |
| 5 mg | 120 | 65% | 30% | 46% | <1% | no |
| 7 mg | 120 | 73% | 32% | 44% | <1% | no |
| 10 mg | 120 | 80% | 41% | 52% | <1% | no |
| 20 mg | 120 | 90% | 43% | 48% | <1% | no |
| 40 mg | 120 | 94% | 43% | 46% | <1% | no |
| 1 mg | 130 | 31% | 11% | 35% | <1% | no |
| 3 mg | 130 | 73% | 35% | 48% | <1% | no |
| 5 mg | 130 | 87% | 46% | 53% | <1% | no |
| 7 mg | 130 | 92% | 50% | 55% | <1% | no |
| 10 mg | 130 | 94% | 49% | 52% | <1% | no |
| 20 mg | 130 | 96% | 54% | 57% | <1% | no |
| 40 mg | 130 | 97% | 54% | 55% | <1% | yes |
| 1 mg | 140 | 72% | 30% | 42% | <1% | no |
| 3 mg | 140 | 91% | 46% | 51% | <1% | no |
| 5 mg | 140 | 95% | 53% | 56% | <1% | no |
| 7 mg | 140 | 96% | 53% | 55% | <1% | no |
| 10 mg | 140 | 98% | 55% | 56% | <1% | no |
| 20 mg | 140 | 98% | 56% | 57% | <1% | no |
| 40 mg | 140 | 99% | 56% | 56% | <1% | yes |
| 1 mg | 150 | 94% | 44% | 46% | <1% | no |
| 3 mg | 150 | 96% | 52% | 54% | <1% | no |
| 5 mg | 150 | 98% | 56% | 57% | <1% | no |
| 7 mg | 150 | 98% | 57% | 59% | <1% | no |
| 10 mg | 150 | 98% | 58% | 59% | <1% | yes |
| 20 mg | 150 | 97% | 61% | 63% | <1% | yes |
| 40 mg | 150 | 97% | 61% | 63% | <1% | yes |

### 5.1.4 Amberlite 15^{®} as a catalyst

Furthermore, a strongly acidic, macroreticular resin with sulfonic acid functionality, namely Amberlite 15^{®}, was tested in NMP following GP1, giving similar results as Montmorillonite KSF^{®} catalyst, but at significantly lower reaction temperature (see Table 5 below) and circumventing the formation of black tar.

**Table 5**

| **Catalyst amount** | **Temp. (°C)** | **Reaction time** | **Fructose cons.** | **HMF yield** | **HMF selectivity** | **LA yield** |
|---|---|---|---|---|---|---|
| 10 mg | 100 | 3h | 70% | 50% | 71% | <1% |

### 5.1.5 SiO₂-MgCl₂ as a catalyst

Since the silica gel - magnesium chloride - complex was found to be catalytically active towards carbohydrate dehydratization reactions in acetonitrile (Yasuda, M.; Nakamura, Y.; Matsumoto, J.; Yokoi, H. Shiragami, T. Bull. Chem. Soc. Jpn. 2011, 84, 416-418), we applied these conditions to NMP as a potent solvent for carbohydrates. The conditions, as stated in GP1, were tuned to reach a maximum HMF yield of 26%. However, when using silica gel only without additive to form the active complex, the conversion to HMF dropped to less than 1%. Accompanying, a massive formation of black tar was observed. See e.g. Table 6 below:

**Table 6**

| **Catalyst amount** | **Temp. (°C)** | **Reaction time** | **Fructose cons.** | **HMF yield** | **HMF selectivity** | **LA yield** |
|---|---|---|---|---|---|---|
| 200 mg | 150 | 30 min | 99% | 26% | 26% | 4% |

### 5.1.6 AlCl₃ as acatalyst

To test also a Lewis acid catalyst in the same setup (GP1), freshly sublimed aluminium trichloride and NMP were chosen as representative candidates. The catalyst showed similar properties as the Amberlite 15^{®}, but is prone to hydrolysis and therefore lacks applicability in a repeated or continuous conversion. Additionally, also here massive formation of black tar was monitored. See e.g., Table 7 below:

**Table 7**

| **Catalyst amount** | **Temp. (°C)** | **Reaction time** | **Fructose cons.** | **HMF yield** | **HMF selectivity** | **LA yield** |
|---|---|---|---|---|---|---|
| 10 mg | 1000 | 3h | 100% | 50% | 50% | <1% |

### 5.1.7 SILPs in combination with chromium-(II)-chloride as catalyst

The additional influence of silica-supported ionic liquid particles, together with chromium-(II)-chloride in NMP was investigated applying GP1. After 20 min, a respectable yield of almost 50% was found, which unfortunately did not improve with longer reaction time. At short reaction times, interconversion of fructose to glucose was observed.

See e.g. Table 8 below:

**Table 8**

| **Temp. (°C)** | **Reaction time** | **Fructose cons.** | **Glucose IC** | **HMF yield** | **HMF selectivity** | **LA yield** |
|---|---|---|---|---|---|---|
| 120 | 5 min | 85% | 5% | 39% | 46% | <1% |
| 120 | 10 min | 94% | 3% | 45% | 48% | <1% |
| 120 | 15 min | 99% | 1% | 44% | 45% | <1% |
| 120 | 20 min | 97% | 2% | 49% | 51% | <1% |
| 120 | 25 min | 97% | 1% | 47% | 48% | <1% |
| 120 | 30 min | 98% | < 1% | 49% | 50% | <1% |
| 120 | 45 min | 99% | < 1% | 48% | 49% | <1% |
| 120 | 1 h | 99% | < 1% | 52% | 52% | <1% |

### 5.2 Experiments performed in the microwave

### 5.2.1 Sulphuric acid as a catalyst

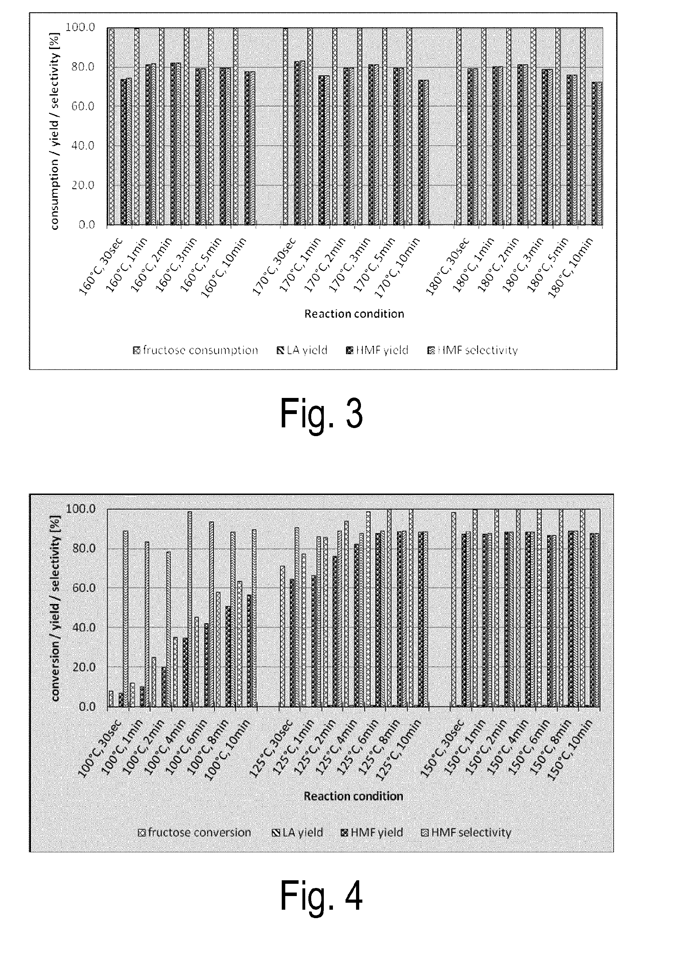

To precisely control heating, steady-state and cooling phases during the dehydratization reactions, also microwave-assisted heating was applied. The samples were prepared as mentioned in GP2 using NMP as a solvent. No formation of black tar was observed under the defined reaction conditions. Furthermore, a trend towards lower reaction time and higher temperature was clearly visible, leading to full fructose conversion and a maximum HMF yield of 83%. Results are set out in Fig. 2 and Fig. 3.

### 5.2.2 Hydrochloric acid as a catalyst

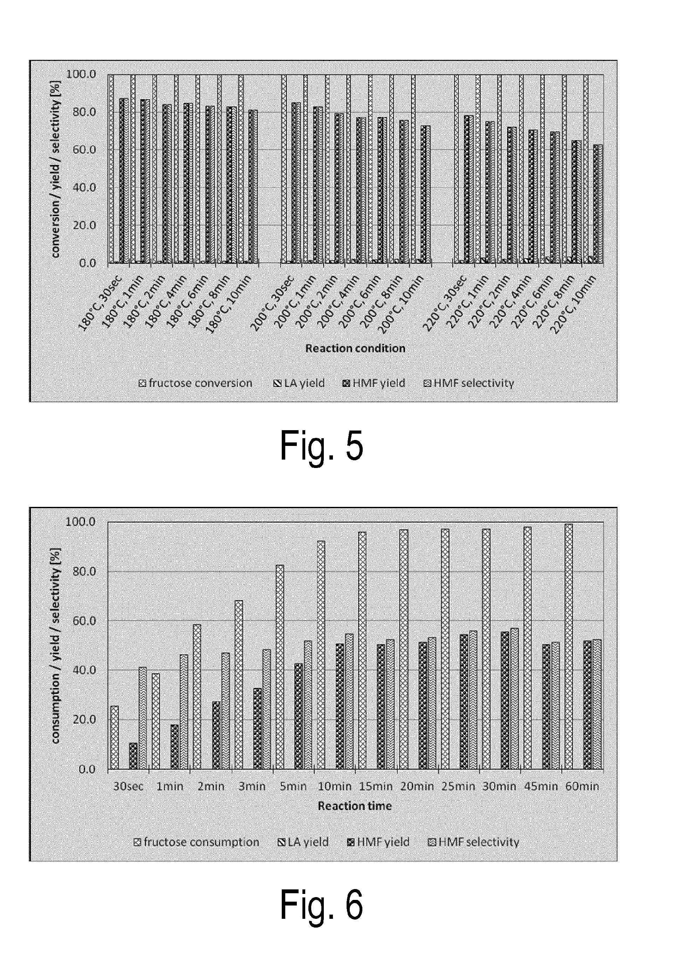

Fructose dehydratization in a stopped flow microwave using NMP was performed according to GP3. The progress of starting material consumption and product/by-product formation follows a strict trend, leading to full fructose conversion and a maximum HMF yield of 89%. Results see in Fig. 4 and Fig. 5.

### 5.2.3 Montmorillonite KSF^{®} as a catalyst

To take advantage of the rapid heating and cooling phases as well as the very accurate temperature regulation directly inside the reaction vial, also heterogeneous Montmorillonite KSF^{®}-containing reaction mixtures with NMP were exposed to microwave irradiation as described in GP2. Reaction time was adjusted to 5 min. Although the HMF yields were only moderate, no black tar formation was present, see e.g. Table 9 below:

**Table 9**

| **Catalyst amount** | **Temp. (°C)** | **Fructose cons** | **HMF yield** | **HMF selectivity** | **LA yield** | **Black tar** |
|---|---|---|---|---|---|---|
| 5 mg | 150 | 51% | 20% | 39% | <1% | no |
| 7 mg | 150 | 61% | 26% | 43% | <1% | no |
| 10 mg | 150 | 64% | 30% | 46% | <1% | no |
| 15 mg | 150 | 76% | 38% | 50% | <1% | no |
| 20 mg | 150 | 82% | 43% | 52% | <1% | no |

To evaluate also the reaction time, the best conditions (20 mg catalyst) were investigated in a time screening. Results see in Fig. 6.

### 5.3 Experiments performed in flow (continous process)

### 5.3.1 Sulphuric acid as a catalyst

Fructose (10% m/v) and concentrated sulphuric acid (1% v/v) were dissolved in NMP and supplied to the PFA capillary continuous flow reactor setup. Samples were prepared by passing 18 mL of solution through the reactor to the waste and collecting subsequent 10 mL of product solution into glass vials, both at 150°C as target temperature. Results are set out in Table 10 below:

**Table 10**

| **Flow rate (mL/min)** | **Residence time (min)** | **Fructose cons.** | **HMF yield** | **HMF selectivity** | **LA yield** |
|---|---|---|---|---|---|
| 0.8 | 20 | 100% | 74% | 74% | <1% |
| 1.6 | 10 | 100% | 75% | 75% | <1% |
| 3.2 | 5 | 100% | 76% | 76% | <1% |

Formation of black, insoluble polymers and humins were not observed under the applied conditions.

### 5.3.2 Hydrochloric acid as a catalyst

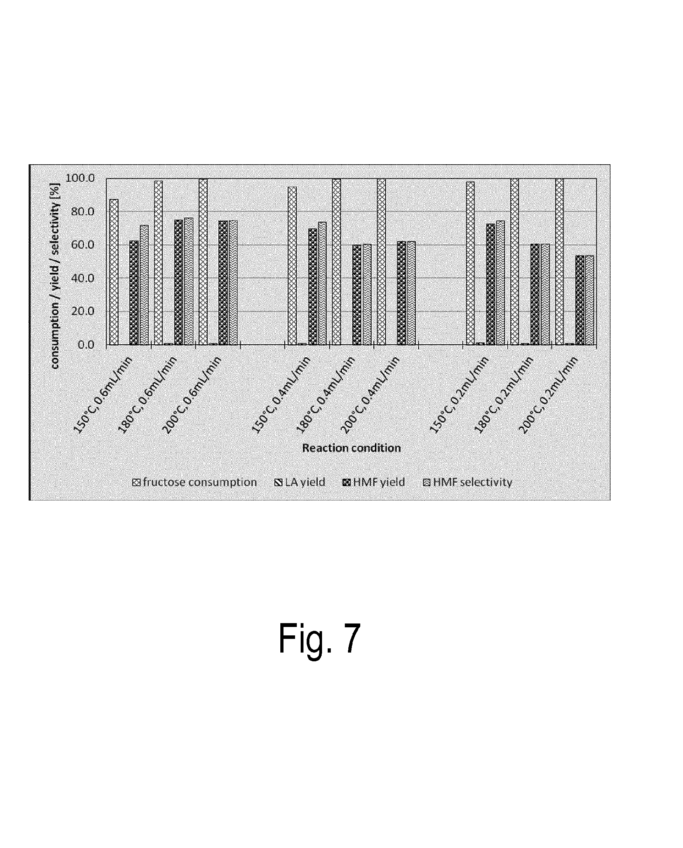

Finally, dehydratization properties of hydrochloric acid in NMP under continuous flow conditions were evaluated according to GP4. Maximum HMF yield of 75% could be achieved at 180°C and 0.6 mL/min, giving a product selectivity of 76%. Levulinic acid yield was mostly below 1%. Results are set out in Fig. 7.

## Claims

1. A process for the production of furan derivatives from carbohydrates in the presence of an acidic catalyst, **characterized in that** N-methylpyrrolidone is used as a solvent.

2. The process according to claim 1, **characterized in that** the process is carried out as a batch process.

3. The process according to claim 1, **characterized in that** the process is carried out as a a continuous process.

4. The process according to any one of claims 1 to 3, **characterized in that** the process is carried out under microwave irridation.

5. The process according to any one of claims 1 to 4, **characterized in that** the furan derivative is 5-hydroxymethylfurfural.

6. The process according to any one of claims 1 to 5, **characterized in that** the carbohydrate is a sugar.

7. The process according to claim 6, **characterized in that** the carbohydrate is fructose.

8. The process according to any one of claims 1 to 7, **characterized in that** N-methylpyrrolidone is used as the reaction solvent.

9. The process according to any one of claims 1 to 7, **characterized in that** N-methylpyrrolidone is used as a reaction co-solvent.

10. The process according to any one of claims 1 to 9, **characterized in that** the acid catalyst is homogenous.

11. The process according to claim 10, **characterized in that** acid catalyst is sulphuric acid or hydrochloric acid.

12. The process according to any one of claims 1 to 9, **characterized in that** the acidic catalyst is heterogenous.

13. The process according to claim 12, **characterized in that** the acidic catalyst is a Montmorillonite, in particular Montmorillonite KSF^{®}, or an Amberlite, in particular Amberlite 15^{®}.

14. The process according to any one of claims 1 to 13, **characterized in that** the reaction is carried out at a temperature from 100 to 220°C, preferably from 125 to 200°C, more preferably from 140°C to 170°C.

15. The process according to any one of claims 1 to 14, **characterized in that** the reaction time is from 30 seconds to 20 minutes, preferably from 1 minute to 10 minutes, more preferably from 2 to 6 minutes.
